# EUROPEAN PATENT APPLICATION

(11) **EP 4 506 324 A1**
(43) Date of publication of application: **12.02.2025**
(21) Application number: 24154735.5
(22) Date of filing: 30.01.2024
(51) Int. Cl.: C07B 59/00, C07C 253/30, C07C 255/42

(54) **REACTION PROCEDURE FOR PRODUCING [F-18]FEONM WITH NO TOXIC SUBSTANCE USED**

(30) Priority: 11.08.2023 TW 112130367
(71) Applicant: National Atomic Research Institute, Taoyuan 32546 (TW)
(72) Inventor: Tu, Yean-Hung, 32546 Taoyuan (TW); Chen, Jenn-Tzong, 32546 Taoyuan (TW); Farn, Shiou-Shiow, 32546 Taoyuan (TW)
(74) Representative: Lang, Christian

(57) **Abstract**

[F-18]FEONM is produced with no toxic substance used. At first, HF[F-18] is transformed into KF[F-18]. A precursor of TEONM is generated after evaporation. An amount of the precursor TEONM is taken. In the presence of both of a phase transfer catalyst and a non-proton phase transfer solvent, a raw product is obtained through a counter-ion phase-transfer radiofluorination. After purifying the raw product through solid-phase extraction, a product of [F-18]FEONM is obtained. [F-18]FEONM is a positron imaging agent used as a new label. Oxolane(THF) or dimethylsulfoxide(DMSO) is used as a non-toxic proton phase transfer solvent for production. The [F-18]FEONM drug has a half-life of only 2 hours. Since no toxic compound is participated in the production and the yield reaches more than 50%, it accelerates the development of PET imaging agent.

## Description

### Technical Field of the invention

The present invention relates to a reaction procedure of [F-18]FEONM; more particularly, to a positron emission tomography (PET) radiopharmaceutical, where a synthesis procedure using non-toxic chemical substance is achieved.

### Description of the Related Arts

Microtubule is the largest protein subunit in cytoskeleton stabilized by tau protein. Hence, the uptake of a tau PET agent of 2 - ( 1 - { 6 - [ ( 2 - 2' - [ F - 18 ] fluoroethoxyethyl ) ( methyl ) amino ] - 2 - naphthyl } - ethylene ) malononitrile ([F-18]FEONM) represents the state of microtubule stability between spheroid and adherent mesenchymal stem cell.

Although nuclear medicines have great market potential for development, patents related to nuclear medicine are quite few, not to mention those for non-toxic chemical reaction procedure. Hence, the prior art does not fulfill all users' requests on actual use.

### Summary of the Invention

The main purpose of the present invention is to use dimethyl sulfoxide or tetrahydrofuran (oxolane, THF) as an aprotic phase-transfer solvent in a production procedure of counter-ion phase-transfer radiofluorination, where an [F-18]FEONM drug with a half-life of only 2 hours is produced; and, since no toxic compound is participated in the production procedure with a yield reaching more than 50%, it accelerates the development of a Positron Emission Tomography (PET) imaging agent.

To achieve the above purpose, the present invention is a reaction procedure for producing [F-18]FEONM with no toxic substance used, comprising steps of: (a) converting [fluorine-18]hydrogen fluoride (HF[F-18]) to [fluorine-18]potassium fluoride (KF[F-18]); and, after evaporating KF[F-18], obtaining a precursor of TEONM, where the precursor of TEONM is a tosylated precursor; (b) obtaining the precursor of TEONM with a phase-transfer catalyst and an aprotic phase-transfer solvent to process a counter-ion phase-transfer radiofluorination at a reaction temperature of 95~110 degrees Celsius (°C) to obtain a raw product; and (c) processing the raw product through a solid-phase extraction with an alumina column and then removing unreacted ions of F-18 to obtain a product of 2 - ( 1 - { 6 - [ ( 2 - 2' - [ F - 18 ] fluoroethoxyethyl ) ( methyl ) amino ] - 2 - naphthyl } - ethylidene ) malononitrile ([F-18]FEONM) through purification, where [F-18]FEONM has an ethyloxy functional group added to an F-18 end of [F-18]FEONM to increase hydrogen bonding to an overall structure of [F-18]FEONM compared with [F-18]FDDNP.

### Brief Description of the Drawings

The present invention will be better understood from the following detailed description of the preferred embodiment according to the present invention, taken in conjunction with the accompanying drawings, in which
- FIG.1: is the flow view showing the preferred embodiment according to the present invention;
- FIG.2: is the view showing the yields of [F-18]FEONM at the reaction temperature of 95 degrees Celsius (°C);and
- FIG.3: is the view showing the [F-18]FEONM Radiofluorination Process Reaction Yield.

### Description of the Preferred Embodiment

The following description of the preferred embodiment is provided to understand the features and the structures of the present invention.

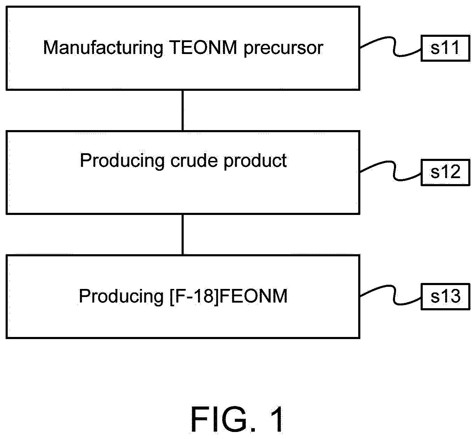

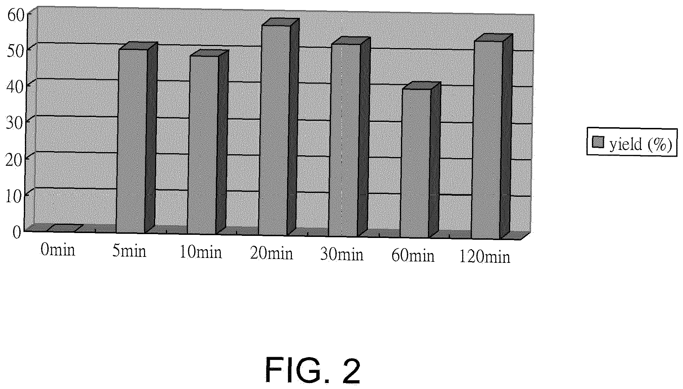

Please refer to FIG.1 and FIG.2, which are a flow view showing a preferred embodiment according to the present invention; and a view showing yields of [F-18]FEONM at a reaction temperature of 95°C. As shown in the figures, the present invention is a reaction procedure for producing [F-18]FEONM with no toxic substance used, comprising the following steps:
(a) Manufacturing TEONM precursor 11: [Fluorine-18]hydrogen fluoride (HF[F-18]) is obtained to be converted to [fluorine-18]potassium fluoride (KF[F-18]); and, after evaporating KF[F-18], a precursor of TEONM is obtained. Therein, the precursor of TEONM is a tosylated precursor.
(b) Producing crude product 12: The precursor of TEONM is obtained with a phase-transfer catalyst and an aprotic phase-transfer solvent to process a counter-ion phase-transfer radiofluorination at a reaction temperature of 95~110°C for obtaining a raw product.
(c) Producing [F-18]FEONM 13: The raw product is processed through a solid-phase extraction with an alumina column and then unreacted ions of F-18 is removed for obtaining a product of 2 - ( 1 - { 6 - [ ( 2 - 2' - [ F - 18 ] fluoroethoxyethyl ) ( methyl ) amino ] - 2 - naphthyl } - ethylidene ) malononitrile ([F-18]FEONM) through purification. Therein, [F-18]FEONM has an ethyloxy functional group added to an F-18 end of [F-18]FEONM to increase hydrogen bonding to an overall structure of [F-18]FEONM whose overall structure is as follows:

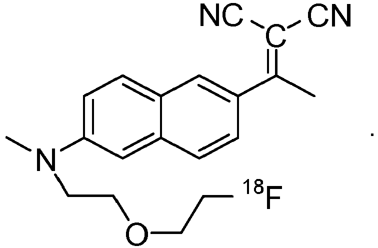

Thus, a novel reaction procedure for producing [F-18]FEONM with no toxic substance used is obtained.

On using the present invention, [F-18]FEONM proposed by the present invention is obtained through synthesis with 1 ~ 7 milli-grams (mg) of the precursor TEONM (tosylated precursor) dissolved in 1 milli-liter (mL) of acetonitrile (Merck DNA synthesis grade) in a reactor made of carboxylated glass, where [2.2.2]cryptate (Crypt-222) or potassium carbonate (K₂CO₃) is used as a phase-transfer catalyst. Since the reaction requires an environment free of polar solvent to maintain the reactivity of F-18 ions, all reactant mixtures must be pumped dry in order to remove polar solvents such as water prior to adding precursors. Aprotic solvents like acetonitrile and dimethylformamide (DMF) help evaporation. The vacuum status of the reactor is an indication of the remainer of the aprotic phase-transfer solvent in the reactor. The pressure for radiofluorination is maintained at 200-240 kilopascals (kPa) to obtain a higher production yield; the reaction time is 5 minutes; and the temperature is controlled at 110°C. A filter of alumina column is installed on a purification unit to remove unreacted F-18 ions, and the first filter absorbs more than 90% of the F ions. The above procedure is operated in an automatic synthesizer shielded with lead glass.

In a state-of-use, [F-18]FEONM uses dimethyl sulfoxide (DMSO) to reach a production yield of the aprotic phase-transfer solvent higher than 50% in the counter-ion phase-transfer radiofluorination.

In a state-of-use, [F-18]FEONM uses acetonitrile to reach a production yield of the aprotic phase-transfer solvent higher than 80% in the counter-ion phase-transfer radiofluorination.

In a state-of-use, [F-18]FEONM uses tetrahydrofuran (oxolane, THF) as the aprotic phase-transfer solvent in the counter-ion phase-transfer radiofluorination.

In a state-of-use, after a calculation of molecular docking force field, [F-18]FEONM is applied in imaging prion-protein-related oligomer quaternary (6RHY tetramer) of Alzheimer's disease beta(β)-amyloid plaque (ABeta).

The following states-of-use are only examples to understand the details and contents of the present invention, but not to limit the scope of patent of the present invention.

### <Material of HF[F-18]>

HF[F-18] is obtained from a liquid target filled with [O-18] (Oxygen-18) water, which is irradiated by a proton beam of a cyclotron, and then transported to a lead chamber through a target-material deliverer.

### <Preparation of [F-18]FEONM>

KF[F-18] is obtained by converting HF[F-18] at the present of potassium carbonate. A phase-transfer catalyst of Crypt-222 is adopted; an aprotic phase-transfer solvent of dimethyl sulfoxide is obtained, which is not a toxic chemical and is used as a phase-transfer reagent in preparation. A precursor of TEONM for radiofluorination is generated after evaporating KF[F-18] for preventing hydration of F-18 compound. A counter-ion phase-transfer radiofluorination (substitution) on the precursor of TEONM is processed at high temperature to obtain a raw product. A solid-phase extraction is performed by passing the radiofluorinated raw product through an alumina column to remove unreacted F-18 ions. The raw product obtained after removing the unreacted ions of F-18 is pumped into a stainless-steel loop, and then injected into a diphenyl high-pressure column for separation and purification. After eluting the precursor of TEONM from the raw product by using ethanol as a mobile phase, a final product of [F-18]FEONM is obtained and flown through a sterilized membrane filter to be collected in a pyrogen-free bottle.

A reaction kinetic experiment is conducted to each key step of reactions for [F-18]FEONM. The key step is the counter-ion phase-transfer radiofluorination. The counter-ion phase-transfer radiofluorination is the key reaction for generating [F-18]FEONM, although being a one-step reaction. After converting HF[F-18] to HK[F-18] in the presence of both of a phase transfer catalyst and a aprotic phase-transfer solvent, the kinetics are studied through the spiking of the reactor.

The same results are obtained with the increases in temperature for the counter-ion phase-transfer radiofluorination of [F-18]FEONM, which shows an obedience to basic Arrhenius law. However, with the amount of 3mg of the precursor of TEONM and the aprotic phase-transfer solvent of dimethylsulfoxide for reaction times of 5, 10, 20, 30, 60, and 120 minutes, 34.54%, 47.14%, 52.23%, 62.48%, 63.78%, and 65.35% of fluoride are generated, respectively. Even if the temperature for the counter-ion phase transfer radiofluorination is 95°C, the radiofluorination yields of [F-18]FEONM obtained at the reaction temperature of 95°C are the same as those shown in FIG.2.

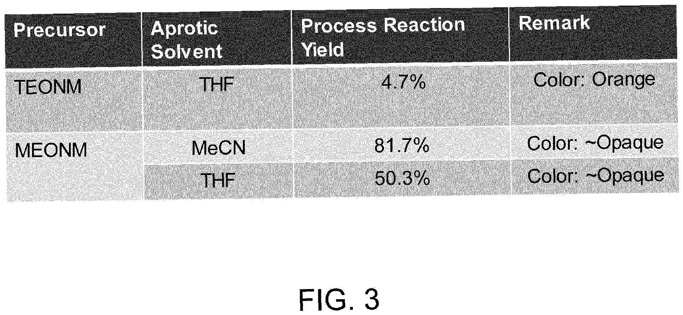

Using THF as a non-proton phase transfer solvent, the yield can reach 50%, as shown in Fig. 3 [F-18]FEONM Radiofluorination Process Reaction Yield.

When the isothermal radiofluorination of [F-18]FEONM uses an amount of 1~7mg of the precursor of TEONM,different fluorination yields are obtained with different concentrations of the precursor of TEONM. The best route for an isothermal irreversible endothermic reaction is the radiofluorination of [F-18]FEONM. At temperatures slightly below its maximum allowed temperature of 95°C, the curve of conversion to reaction temperature is linear. Those for the other reversible endothermic radiofluorination are also straight lines, but the track of all maximum rates form a curve for different states of the reversible exothermic radiofluorination.

The counter-ion phase transfer radiofluorination is processed to the precursor of TEONM for generating the kinetic curve of yield to reaction time, which shows that the reaction temperature is one of the key parameters for obtaining high-yield and high-purity radiofluorinated product of [F-18]FEONM. The radiofluorination of [F-18]FEONM has the same outcome because of its similar steric effect on the nucleophilic substitutional fluorination of these two molecules.

The amount of the precursor of TEONM is another key factor in the kinetic procedure of developing [F-18]FEONM with the radiofluorinated precursor of TEONM. When the chemical amount of the precursor is less than one billion times of F ions, even if the reaction temperature is high enough, the radiofluorination hardly occurs; but a breakthrough may be achieved due to the development of a new technology. More than 90% of the F ions replace leaving groups, when the amount of the precursor of TEONM exceeds 7 billion times of the F ions. The radiofluorination of [F-18]FEONM in the device is an irreversible endothermic reaction.

According to Arrhenius law, on reaching the activation energy, the reaction rate increases with temperature. As the temperature of radiofluorination increases with a constant precursor concentration, the reaction follows Arrhenius law. However, when the amount of the precursor is less than 1mg, the yield does not conform to the Arrhenius law. In these radiofluorination reactions, as compared to that using up to 7mg of the precursor, such a big difference to that using less than 1mg of the precursor is owing to the nanogram-scale stoichiometry of F-18 ion. A billion times more of the precursor as compared to F-18 ions create a radiochemical precursor gap between radiofluorination world and Arrhenius law. Although, according to Arrhenius law, the radiofluorination with the addition of carrier makes the zero interstitial-amount of the precursor. But the specific activity of [F-18]FEONM is very low, because a large amount of [F-19]FEONM cannot be removed by chemical method.

### <Discovery in application>

A force-field analysis is processed by a molecular simulation software (Discovery Studio 2021) with external artificial intelligence (Al) function; and an interaction energy between oligomer quaternary of Alzheimer's disease-related β-amyloid plaque and molecular structural imaging is calculated to verify the interaction force and the imaging result of positron emission tomography (PET) of the Tg2576 β-amyloid plaque transgenic mouse. The ratio of [F-18]FEONM PET agent to Alzheimer's disease β-amyloid plaque oligomer quaternary are 1.6~1.7 times that of [F-18]FDDNP to [F-18]T807.

The interaction force of [F-18]FDDNP on β-amyloid plaque is close to that of [F-18]T807 and [F-18]JNJ311. The main reason is that [F-18]FEONM has more ethyl oxygen functional groups than [F-18]FDDNP, which increases the hydrogen bond interaction in the overall structure. Hence, the force field on the protein quaternary is transferred; the original malononitrile of biomotif force groups of [F -18]FDDNP are transferred to F atoms of [F-18]FEONM; and the F-18 atoms no longer only play the role of emitting positrons in the molecules of [F-18]FDDNP, but also act as biomotif force bases in the molecules of [F-18]FEONM as becoming the center of force field.

The increased hydrogen (H) bond uses the F-atom force of [F-18]FEONM on the 37th amino acid glycine of C-segment peptide chain of the β-amyloid plaque quaternary as the main force with a distance of 2.69A. In addition, its carbon chain acts on the 35th amino acid methionine (methionine) of the C-segment peptide chain as well, which is another H bond increasing the force of [F-18]FEONM and quaternary peptide of β-amyloid plaque with a distance of 3.03A. As compared with the existing methods aimed at removing β-amyloid plaque of Alzheimer's disease, the present invention improves 27% of the cognitive function on the degeneration of cognition and neurodegeneration of early Alzheimer's disease patient, yet its clinical effect on dementia is less than 1% of no significance. Therefore, the [F-18]FEONM contrast agent proposed in the present invention mainly acts on the entanglement of proteins directly related to brain cells such as intracellular tau protein, and the β-amyloid plaque oligomer quaternary that damages the cell membrane of brain nerve cells as leading to brain cell death. Regarding the effect on protein variation directly associated with dementia, the present invention is of help to evaluate the efficacy of developing Alzheimer's dementia drug and shorten the time for developing new drug.

To sum up, the present invention is a reaction procedure for producing [F-18]FEONM with no toxic substance used, where a PET agent of [F-18]FEONM is obtained as a new label and its synthesis production procedure is achieved by using non-toxic chemical substance.

The preferred embodiment herein disclosed is not intended to unnecessarily limit the scope of the invention. Therefore, simple modifications or variations belonging to the equivalent of the scope of the claims and the instructions disclosed herein for a patent are all within the scope of the present invention.

## Claims

1. A method of producing [F-18]FEONM with no toxic substance used, comprising steps of:
(a) converting [Fluorine-18]hydrogen fluoride (HF[F-18]) to [fluorine-18]potassium fluoride (KF[F-18]); and, after evaporating KF[F-18], obtaining a precursor of TEONM,
wherein said precursor of TEONM is a tosylated precursor;
(b) obtaining said precursor of TEONM with a phase-transfer catalyst and an aprotic phase-transfer solvent to process a counter-ion phase-transfer radiofluorination at a reaction temperature of 95~110 degrees Celsius (°C) to obtain a raw product; and
(c) processing said raw product through a solid-phase extraction with an alumina column and then removing unreacted ions of F-18 to obtain a product of 2 - ( 1 - { 6 - [ ( 2 - 2' - [ F - 18 ] fluoroethoxyethyl ) ( methyl ) amino ] - 2 - naphthyl } - ethylidene ) malononitrile ([F-18]FEONM) through purification,
wherein [F-18]FEONM has an ethyloxy functional group added to an F-18 end of [F-18]FEONM to increase hydrogen bonding to an overall structure of [F-18]FEONM whose overall structure is as follows:

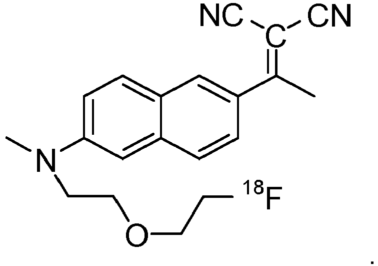

2. The method according to claim 1,
wherein said phase-transfer catalyst is selected from a group consisting of [2.2.2] cryptate (Crypt-222); and potassium carbonate (K₂CO₃).

3. The method according to claim 1,
wherein, in said counter-ion phase-transfer radiofluorination of [F-18]FEONM using dimethyl sulfoxide (DMSO), said aprotic phase-transfer solvent has a production yield more than 50 percent (%).

4. The method according to claim 1,
wherein, in the counter-ion phase-transfer radiofluorination of [F-18]FEONM using acetonitrile, said aprotic phase-transfer solvent has a production yield more than 80%.

5. The method according to claim 1,
wherein [F-18]FEONM obtains tetrahydrofuran (oxolane, THF) as said aprotic phase-transfer solvent in said counter-ion phase-transfer radiofluorination.

6. The method according to claim 1,
wherein, after a calculation of molecular docking force field, [F-18]FEONM involves in imaging prion-protein-related oligomer quaternary (6RHY tetramer) of Alzheimer's disease beta amyloid plaque (ABeta).

7. The method according to claim 1,
wherein said counter-ion phase-transfer radiofluorination has a pressure of 200~240 kilopascals (kPa) and a reaction time of 5 minutes.

8. The method according to claim 1,
wherein, in step (b), an amount of said precursor of TEONM is 1~7 milli-grams (mg).

9. The method according to claim 1,
wherein, in step (c), said raw product obtained after removing unreacted ions of F-18 is injected into a diphenyl high-pressure column to process separation and purification; ethanol in a mobile phase is obtained to elute said precursor of TEONM from said raw product; and, after filtration and sterilization, [F-18]FEONM is obtained.
